# EUROPEAN PATENT SPECIFICATION

(11) **EP 4 045 143 B1**
(45) Date of publication and mention of the grant of the patent: **16.04.2025**
(21) Application number: 20876798.8
(22) Date of filing: 13.10.2020
(51) Int. Cl.: A61K 9/107, A61K 31/506, A61K 9/14, A61K 9/20

(54) **BOSENTAN MONOHYDRATE LOADED LIQUID SELF NANOEMULSIFYING DRUG CARRIER SYSTEM AND TABLET FORMULATION**
MIT BOSENTAN-MONOHYDRAT BELADENES FLÜSSIGES SELBST-NANOEMULGIERENDES ARZNEIMITTELTRÄGERSYSTEM UND TABLETTENFORMULIERUNG
SYSTÈME DE VECTEUR DE MÉDICAMENT AUTONANOÉMULSIFIANT LIQUIDE CHARGÉ DE BOSENTAN MONOHYDRATÉ ET FORMULATION DE COMPRIMÉ

(30) Priority: 14.10.2019 TR 201915787
(43) Date of publication of application: 24.08.2022
(73) Proprietor: Gazi University, Ankara (TR)
(72) Inventor: TEKSIN, Zeynep Safak, Yenimahalle/Ankara (TR); YILMAZ USTA, Duygu, Yenimahalle/Ankara (TR)
(74) Representative: Bulut, Pinar
(86) International application number: PCT/TR2020/050945
(87) International publication number: WO 2021/076081

(56) References cited:
- WO-A1-2011/048493
- WO-A1-2012/139736
- WO-A1-2015/100406
- WO-A1-2016/124966
- PANIGRAHI KAHNU CHARAN ET AL: "QBD-based systematic development of Bosentan SNEDDS: Formulation, characterization and pharmacokinetic assessment", JOURNAL OF DRUG DELIVERY SCIENCE AND TECHNOLOGY, vol. 47, 1 October 2018 (2018-10-01), FR, pages 31 - 42, XP093087747, ISSN: 1773-2247, DOI: 10.1016/j.jddst.2018.06.021
- VADLAMUDI HARINI CHOWDARY ET AL: "In vitro characterization studies of self-microemulsified bosentan systems", JOURNAL DRUG DEVELOPMENT AND INDUSTRIAL PHARMACY, vol. 43, no. 6, 16 February 2017 (2017-02-16), US, pages 989 - 995, XP093087862, ISSN: 0363-9045, DOI: 10.1080/03639045.2017.1287720
- SUHAS G. GUMASTE ET AL: "Development of Solid SEDDS, V: Compaction and Drug Release Properties of Tablets Prepared by Adsorbing Lipid-Based Formulations onto Neusilin® US2", ENVIRONMENTAL SCIENCE AND POLLUTION RESEARCH, vol. 30, no. 12, 25 June 2013 (2013-06-25), Berlin/Heidelberg, pages 3186 - 3199, XP055704836, ISSN: 0724-8741, DOI: 10.1007/s11095-013-1106-4
- GUMASTE SUHAS G. ET AL: "Development of Solid SEDDS, IV: Effect of Adsorbed Lipid and Surfactant on Tableting Properties and Surface Structures of Different Silicates", ENVIRONMENTAL SCIENCE AND POLLUTION RESEARCH, vol. 30, no. 12, 1 December 2013 (2013-12-01), Berlin/Heidelberg, pages 3170 - 3185, XP055812741, ISSN: 0724-8741, Retrieved from the Internet <URL:https://link.springer.com/content/pdf/10.1007/s11095-013-1114-4.pdf> DOI: 10.1007/s11095-013-1114-4

## Description

### TECHNICAL AREA

It is about the adsorption of liquid SNEDDS containing bosentan monohydrate to an adsorbent carrier by the adsorption technique and the conversion of the powder form into solid tablet form (S-SNEDDS) by direct compression.

### BACKGROUND OF THE INVENTION

While only 8% of the new chemical molecules show high solubility and high permeability, approximately 40% of them show difficulty in formulation due to poor water solubility and fail in drug development studies. Limited solubility due to poor water solubility results in the low bioavailability of the drugs. If the water solubility of BCS Class 2 drugs is increased according to the Biopharmaceutics Classification System (BCS), which has poor water solubility and good permeability, the in vivo activities and bioavailability of the drugs will also increase. Various techniques are used to increase the solubility and bioavailability of drugs that are poorly soluble in water. These are techniques such as cosolvent use, micronization, chemical drug modification, surfactant use, particle size reduction technology, nanoparticle technology, nanocrystalline technology, nanosuspension, self emulsifying lipid-based systems, and solid dispersion technique.

Lipid-based drug delivery systems are one of the most important strategies used to increase the solubility and bioavailability of drugs with low water solubility and lipophilic structure. Depending on the developing technology and requirements, it is developed as different dosage forms such as oily solutions, emulsions, microemulsions, self emulsifying drug carrier systems (SEDDS, SMEDDS, and SNEDDS), and liposomes.

SNEDDS are known as self emulsifying isotropic mixtures containing oil, surfactant, and co-surfactant. In systems that are self nanoemulsifying when combined with the aqueous phase in the gastrointestinal tract, the droplet size is below 100 nm and is used to improve the dissolution and absorption of BCS Class 2 drugs. SNEDDS show a significant increase in dissolution rate, solubility, and permeability, and also they significantly reduce p-gp efflux, first-pass effect through the liver, and intra-individual and inter-individual variability depending on the conditions in the gastrointestinal tract. There are many drugs developed in the form of self emulsifying systems on the market: Neoral^{®}, Vesanoid^{®}, Accutane^{®}, Sandimmune^{®}, Kaletra^{®}, Aptivus^{®}, Norvir^{®}, Fortovase^{®}, Agenerase^{®}, Solufen^{®}, and Lipirex^{®}.

Self emulsifying systems have many advantages; however, the observation of general stability problems in lipid-based systems, leakage from the oily system during packaging and transportation, the possibility of irritation in the gastrointestinal system of the high amount of surfactant used in these systems, the soft gelatin capsule where liquid SEDDS formulations can be filled has disadvantages such as being an expensive technology.

Solid self nanoemulsifying systems (S-SNEDDS) are an alternative approach to overcoming the problems of self emulsifying systems. S-SNEDDS combine the advantages of solid dosage forms, such as low production cost, ease of process control, high stability and reproducibility, better patient compliance, with the increased solubility and bioavailability of SEDDS. **In** addition, S-SEDDS are generally preferred to liquid SEDDS due to its ease of transportation and stability.

There are methods such as adsorption, spray drying, dry emulsion, solid dispersion, melt extrusion on solid carriers to obtain S-SNEDDS, and the prepared S-SNEDDS can then be converted into pellets, tablets, and capsules.

Solid carriers have the ability to adsorbtion the liquid / semi-solid self emulsifying systems. A freely flowing powder mass is obtained by mixing, which is a simple procedure. Later, this free-flowing powder mass is formulated with appropriate excipients and made ready for tablet compression. Solid dosage forms with better content uniformity are obtained with this technique.

Microporous inorganic materials, colloidal inorganic adsorbents with large surface area, crosslinked polymers or nanoparticles can be used as carrier material for the absorption of the self emulsifying system.

Magnesium aluminometasilicate has a large specific surface area, high porosity, high oil adsorption capacity. Magnesium aluminometasilicate has been accepted worldwide to be used in formulations containing antibiotics, lipophilic active ingredients, slightly soluble active ingredients, herbal mixtures, and vitamins. **In** pharmaceutical applications, it is widely used as a carrier and filler to improve the quality of tablets, powders, granules, and capsules, as well as carriers for solid dispersions and self emulsifying drug systems. It is useful in increasing the compressibility and fluidity of powders, increasing the hardness of tablets even at low compression forces, increasing the stability of moisture sensitive active substances, and formulating lipophilic oils or active substances with adsorption capacity.

Bosentan monohydrate is a non-peptide endothelin receptor antagonist, specifically administered orally in the treatment of pulmonary arterial hypertension (PAH). Reference and equivalent products used in the treatment of PAH are available in the pharmaceutical market in the form of 62.5 and 125 mg tablets. In order to observe the therapeutic effect of the products in patients, they should be used at a dose of 62.5 mg twice a day in the first four weeks of treatment and then at a dose of 125 mg twice a day. This treatment requires lifelong drug use. Since it is a very expensive treatment, its economic cost to both patients and society is quite high. Bosentan, a BCS Class 2 drug, has a problem of solubility, bioavailability, and dose-dependent side effects. To solve these problems, it is necessary to increase the bioavailability with appropriate formulation strategies and to reduce the side effects and the amount/number of doses.

Various dosage forms were prepared for bosentan monohydrate such as osmotic controlled tablets, solid dispersion, extended-release matrix tablets prepared with synthetic/natural polymers, mucoadhesive microsphere, extended-release pellet, controlled release system with microspheres prepared with biodegradable polymer mixture, transdermal patch, inhaled controlled release polymeric colloid, fast-dissolving nanosuspension, nanocomposite, self microemulsifying, and self nanoemulsifying systems. Vadlamudi H.C. et al. (Drug Development and Industrial Pharmacy 2017, vol. 43(6), pages 989-995) discloses *in vitro* characterization studies of liquid and solid self-nanoemulsifying bosentan systems. With these systems, it is aimed to increase the solubility/dissolution rate of the active substance and thus increase the treatment efficiency.

In the present invention, it is aimed to combine the advantages of the solid dosage form and conventional systems with a new self nanoemulsifying system. In addition, it is thought that designing the solid dosage S-SNEDDS form of bosentan monohydrate will be more advantageous than the dosage form in the market, considering the advantage of the use of the patient and the reduction of possible pharmacoeconomic treatment costs.

### DESCRIPTION OF THE INVENTION

S-SNEDDS tablets were prepared to increase the in vitro dissolution and bioavailability of bosentan monohydrate, which has a very low water solubility and dissolution rate. In the preparation of SNEDDS, glyceryl monolinoleate (Maisine^{®}) as oil, polyoxyl 40 hydrogenated castor (Cremophor^{®}RH 40) oil as surfactant and caprilocaproyl polyoxyl-8 glycerides (Labrasol^{®}) as cosurfactant were used. Magnesium aluminometasilicate (Neusilin^{®} US2) was used for solidification of the liquid SNEDD system. In the preparation of the solid system, the method of adsorbing on a carrier adsorbent was chosen.

After the adsorption process, the characterization studies of the powder mass were carried out by scanning electron microscopy, X-ray diffraction analysis, differential scanning calorimetry, total pore volume and specific surface area analysis.

Croscarmellose sodium and magnesium stearate were used as excipient for tabletting the powder mass by direct compression, and tablet controls were carried out on the obtained tablets. **In** vitro dissolution studies were carried out by using distilled water environment containing 1% SLS, which is the in vitro dissolution medium recommended by the FDA, and in Fasted State Simulating Intestinal Fluid (FaSSIF) and Fed State Simulating Intestinal Fluid (FeSSIF) biorelevant media. As a result, bosentan monohydrate was successfully loaded into liquid SNEDDS and magnesium aluminomethacilica, the tablets with good powder properties and obtained by direct compression of this powder passed the tablet controls successfully.

| | FaSSIF | FeSSIF |
|---|---|---|
| Sodium taurocholate | 3 mM | 15 mM |
| Lecithin | 0.75 mM | 3.75 mM |
| NaOH (pellet) | 0.174 g | 4.04 g |
| NaH₂PO₄.H₂O | 1.977 g | - |
| Glacial Acetic Acid | - | 8.65 g |
| NaCl | 3.093 g | 11.874 g |
| Distilled water q.b. | 500 mL | 1000 mL |
| pH | 6.50 | 5.00 |
| Osmolality | ~ 270 mOsmol/kg | ~ 670 mOsmol/kg |

The liquid SNEDDS formulation in distilled water containing 1% SLS released more than 80% of the commercial tablet within 15 minutes and all of the bosentan monohydrate within 30 minutes. S-SNEDDS tablet released 87% at the end of 90 minutes. In FaSSIF medium, which mimics biorelevant media, the commercial tablet released approximately 33% of the active substance after 90 minutes, while the liquid SNEDDS formulation loaded with bosentan monohydrate released 96% and S-SNEDDS tablet 79%. In the FeSSIF medium, the commercial tablet released approximately 11% of the active substance after 90 minutes, while the liquid SNEDDS formulation loaded with bosentan monohydrate released 86% and the S-SNEDDS tablet 81%.

The ratio of components in an exemplary liquid SNEDDS formulation is 0.9: 7.2: 0.8 by weight, which is oil: surfactant: cosurfactant. Accordingly, the ratio of glyceryl monolinoleate, polyoxyl 40 hydrogenated castor oil and caprylocaproyl polyoxyl-8 glycerides by weight is 0.9: 7.2: 0.8 (w/w/w).

In the liquid SNEDDS formulation, the particle size is below 50 nm and the polydispersity index (PDI) is less than 0.2. Liquid SNEDDS has a droplet particle size of 17.11 nm and a polydispersity index of 0.180.

1 gram of the liquid SNEDDS formulation contains 30 mg of bosentan monohydrate.

The ratio of the liquid SNEDDS formulation to magnesium aluminometacilicate can be between 1:1 and 1.5:1 (w/w) by weight. Liquid SNEDDS: magnesium aluminometasilicate ratio is preferably 1.25:1 (w/w).

5% croscarmellose sodium (w/w) and 1% magnesium stearate (w/w) were used as excipients in tablet formulation of liquid SNEDDS: magnesium aluminometasilicate powder mass.

### Example 1

### Obtaining Liquid SNEDDS with Ternary Phase Diagram

Solubility studies of bosentan monohydrate in different oil groups have been carried out. Considering the hydrophilic-lipophilic balance values (HLB), the highest solubility glyceryl monolinoleate, polyoxyl 40 hydrogenated castor oil, caprylocaproyl polyoxyl-8 glycerides were chosen as oil, surfactant, and cosurfactant. One type of mixtures were obtained by scanning the surfactant and cosurfactant ratio between 9: 1 to 1: 9. Smix (surfactant: cosurfactant ratio) was determined as 9: 1. The obtained homogeneous mixtures were then mixed with oil in the ratio of 9: 1-1: 9 to create a homogeneous system. These systems were then titrated with water and the boundaries of the ternary phase diagrams containing the nanoemulsion field were determined.

### Optimization of SNEDDS Formulation

Mixtures of different proportions within the confines of the ternary phase diagrams were diluted with 1:250 distilled water to determine whether nanoemulsion was formed. The determined extreme values were evaluated with the Design Expert^{®} program, and the optimum SNEDDS formulation was created and the strength of the formulation was supported by characterization studies. Based on the SNEDDS formulation supported by studies, liquid SNEDDS formulations loaded with bosentan monohydrate were prepared. Different amounts of active substance were tested and mixed with blank formulations and vortexed until homogeneous preparations were obtained. It was then allowed to dissolve completely in a water bath at 37°C for 24 hours, and the resulting bosentan monohydrate loaded SNEDDS formulations were stored at room temperature until use. In this study, 30 mg of bosentan monohydrate was loaded into 1 g of liquid SNEDDS formulation.

### Conversion of SNEEDS Formulation to S-SNEEDS

1 g liquid SNEDDS formulation loaded with 30 mg bosentan monohydrate was added to the magnesium aluminometasilicate selected as an adsorbent in a mortar and turned into a solidified (S-SNEDDS) powder form. For this purpose, three different ratios of bosentan monohydrate loaded liquid SNEDDS: magnesium aluminometasilicate as 1: 1, 1.25: 1, 1.5: 1 were tested. In order to prepare tablets, 5% of the main mass was determined as croscarmellose sodium and 1% magnesium stearate as excipients and mixed in a cubic mixer. After examining the powder flow properties and characterization studies, the ratio of 1: 1 was selected and tablets were obtained by direct compression technique.

### In Vitro Dissolution Studies

The in vitro dissolution study test conditions performed with reference product Tracleer^{®} tablet (expiry date 10/20 and batch number IW067A0401), BOS-loaded liquid SNEDDS formulation and BOS-loaded S-SNEDDS tablet are given below. Dissolution studies were carried out according to the specified experimental conditions. 5 mL of the aliquots were withdrawn at predetermined time intervals, (15, 30, 45, 60, and 90 min) from the dissolution medium and with an equal volume of fresh medium to maintain the total volume. The withdrawn samples were filtered using a 0.45 mm.

Biorelevant media was prepared by dissolving 2.24 g of SIF powder in one liter of pH 6.5 buffer for FaSSIF and by dissolving 11.2 g of SIF powder in one liter of pH 5.0 buffer for FeSSIF.

### Test conditions of in vitro dissolution studies

| | |
|---|---|
| **Method** | USP Apparatus II (Paddle method) |
| **Dissolution media** | % 1 SLS, FaSSIF, FeSSIF |
| **Media volume** | 900 mL |
| **Wavelength** | 220 nm |
| **Speed** | 50 rpm |
| **Temperature** | 37°C±0,5°C |
| **Sampling intervals** | 15, 30, 45, 60 and 90 minutes |
| **Analysis method** | HPLC |
| **Tablet - capsule samples** | 3 |

### Results

The liquid SNEDD system carrying 30 mg of bosentan monohydrate obtained consists of 10.11% glyceryl monolinoleate, 80.90% polyoxyl 40 hydrogenated castor oil, and 8.99% caprilocaproyl polyoxyl-8 glycerides. The ratio of components is about 0.9: 7.2: 0.8 by weight (w/w/w).

The liquid SNEDD system loaded with bosentan monohydrate has a droplet size of 17.11 nm and a polydispersity index of 0.180.

The powder properties of the bosentan monohydrate loaded liquid SNEDDS: magnesium aluminometasilicate 1.25: 1 ratio were found suitable (Table 1).

**Table 1. Powder properties of the 1.25:1 ratio of BOS-loaded liquid SNEDDS: magnesium aluminometasilicate**

| **Bulk Density** | **Tapped Density** | **Angle of Repose** | **Carr's index** | **Hausner's Ratio** |
|---|---|---|---|---|
| 0.36 | 0.44 | 22.6 | 18 | 1.22 |

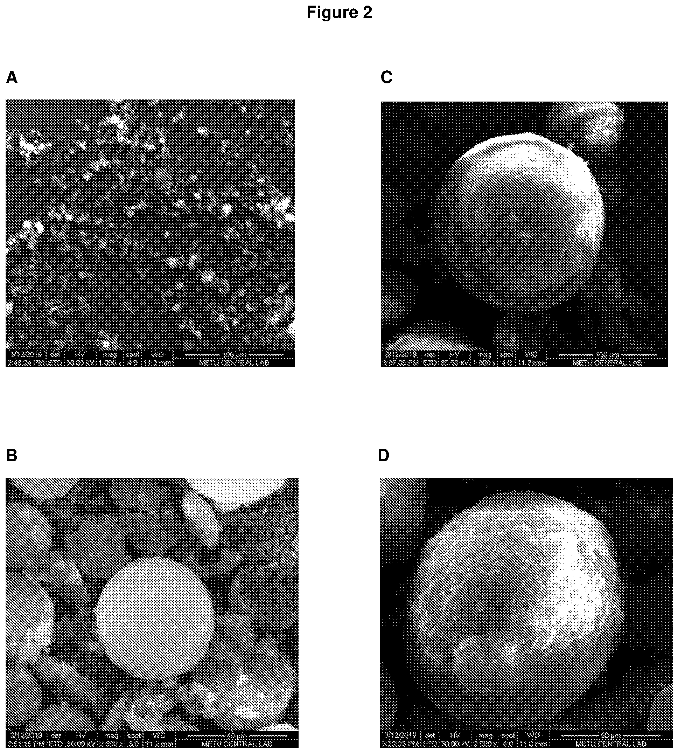

Morphological analyses of bosentan monohydrate, magnesium aluminometasilicate and liquid SNEDDS: magnesium aluminometasilicate (1.25:1) and BOS-loaded liquid SNEDDS: magnesium aluminometasilicate (1.25:1) by SEM were performed. It was observed that the liquid SNEDDS loaded with bosentan monohydrate was successfully adsorbed to the adsorbent and the spherical smooth structure of magnesium aluminometasilicate was not disturbed (Figure 2).

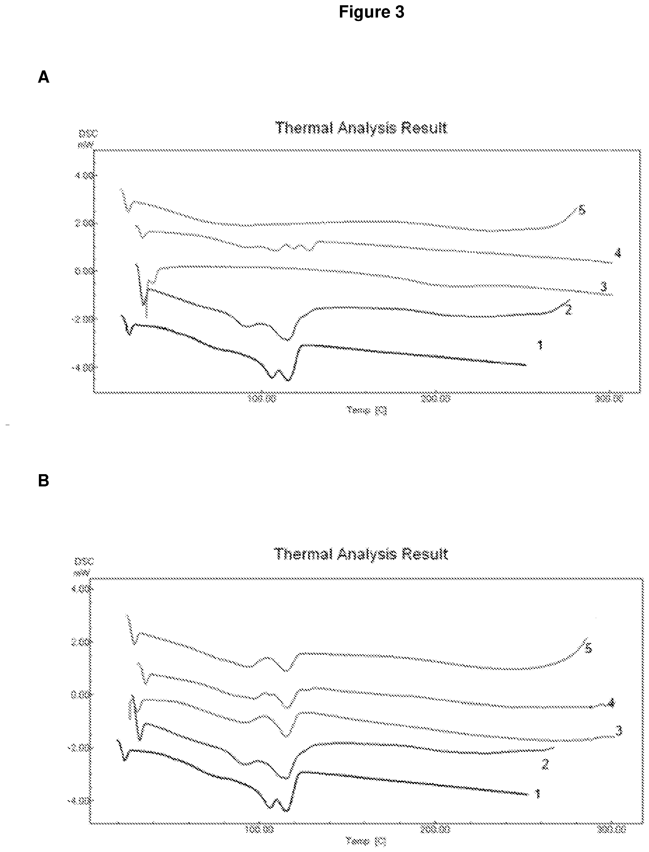

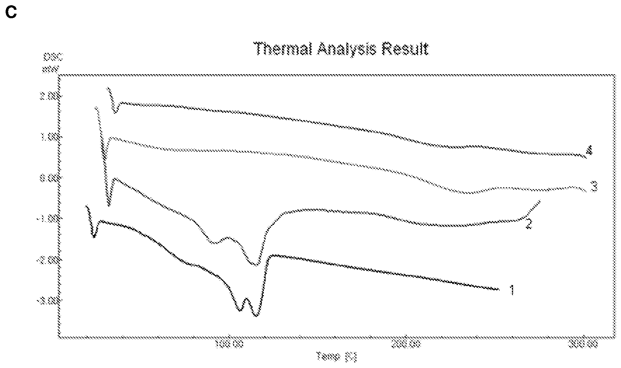

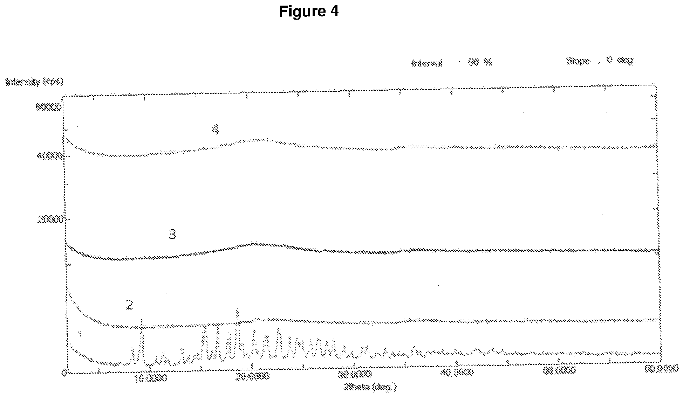

DSC analysis were performed for bosentan monohydrate, tablet excipients, magnesium aluminometasilicate and liquid SNEDDS: magnesium aluminometasilicate (1.25:1) and BOS-loaded liquid SNEDDS: magnesium aluminometasilicate (1.25:1) and bosentan monohydrate is compatible with tablet excipients. It was determined by thermograms that monohydrate loaded liquid SNEDDS was successfully adsorbed to the adsorbent (Figure 3). XRD analysis results were obtained for bosentan monohydrate, magnesium aluminometasilicate, liquid SNEDDS: magnesium aluminometasilicate (1.25:1), and BOS-loaded liquid SNEDDS: magnesium aluminometasilicate (1.25:1). The results shown that BOS-loaded liquid SNEDDS was successfully adsorbed onto adsorban (Figure 4).

Characterization studies performed on the S-SNEDDS tablet were found appropriate (Table 2).

**Table 2. S-SNEDDS tablet characterization**

| **Hardness* (N)** | **Diameter* (mm)** | **Thickness* (mm)** | **Weight variation* (g)** | **Friability (%)** | **Disintegration Time* (min)** | **Content Uniformity (%)** |
|---|---|---|---|---|---|---|
| 153±10 | 11.11±0.001 | 8.91±0.03 | 1.929±0.027 | 0.00093 | 23.74±0.50 | 102.6 |

| | | | | | | |
|---|---|---|---|---|---|---|
| *mean±standart deviation | | | | | | |

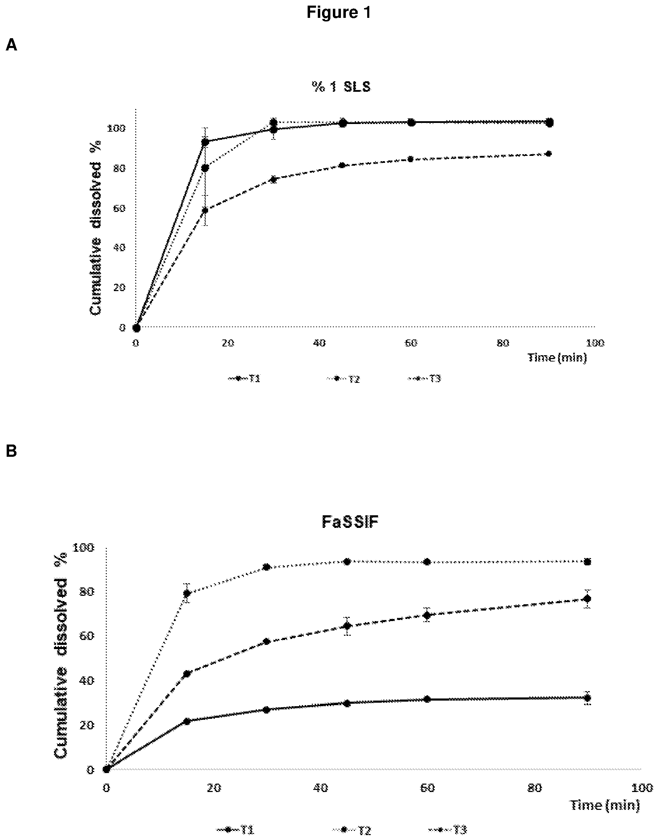

More than 85% of bosentan monohydrate was dissolved after 90 minutes from commercial products and formulations in distilled water containing 1% SLS, as shown in Figure 1A.

As seen in Figure 1B in FaSSIF medium, after 90 minutes from commercial products and formulations, bosentan monohydrate dissolved 33% from the commercial product, while 79% from the S-SNEDDS tablet. BOS-loaded S-SNEDDS tablet showed 2.39-fold more cumulative dissolution than commercial product for bosentan monohydrate.

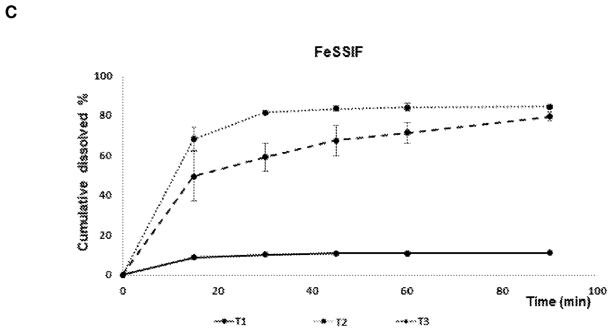

As seen in Figure 1C in FeSSIF medium, after 90 minutes of commercial products and formulations, bosentan monohydrate dissolved 11% from the commercial product and 81% from the S-SNEDDS tablet. BOS-loaded S-SNEDDS tablet showed 7.27-fold more cumulative dissolution than commercial product for bosentan monohydrate.

As a result, the feature of the S-SNEDDS tablet containing Bosentan monohydrate, which is the subject of the invention, is that the liquid SNEDDS containing Bosentan monohydrate as an active substance is adsorbed on magnesium aluminometasilicate. The liquid SNEDD system contains glyceryl monolinoleate as oil, polyoxyl 40 hydrogenated castor oil as a surfactant, and caprilocaproyl polyoxyl-8 glycerides as co-surfactant.

The production method of the S-SNEDDS tablet containing Bosentan monohydrate of the present invention includes the following steps:
- SNEDDS is obtained with glyceryl monolinoleate as oil, polyoxyl 40 hydrogenated castor oil as a surfactant, and caprilocaproyl polyoxyl-8 glycerides as cosurfactant.
- Bosentan monohydrate: glyceryl monolinoleate: Polyoxyl 40 hydrogenated castor oil: Caprilocaproyl polyoxyl-8 glycerides are loaded into the SNEDDS system.
- S-SNEDDS is obtained by loading liquid SNEDDS on magnesium aluminometasilicate as adsorbent.
- S-SNEDDS and tablet excipients are mixed.
- Tablets are obtained by the direct compression method.

### APPLICATION OF THE INVENTION TO INDUSTRY

Liquid SNEDDS is used to prepare solid drug forms by increasing the solubility and dissolution of difficult solutes by converting the powder form to solid tablet form with the technique of adsorbing on an adsorbent carrier (S-SNEDDS).

### Explanation of Figures

**Figure 1****.** Dissolution rate profiles of liquid SNEDDS, S-SNEDDS tablet formulation, and commercial product of bosentan monohydrate (Error bars represent standard deviation).
   A- 1% SLS distilled water medium recommended by FDA,
   B- FaSSIF media,
   C- FeSSIF media,
   T1 = Commercial product (Tracleer); T2 = Liquid SNEDDS; T3 = S-SNEDDS Tablet
**Figure 2****.** SEM images. A- Bosentan monohydrate, B- Magnesium aluminometasilicate, C- Blank liquid SNEDDS: magnesium aluminometasilicate (1.25:1), D- Bosentan monohydrate loaded liquid SNEDDS: magnesium aluminometasilicate (1.25:1)
**Figure 3****.** DSC thermograms.
   A- Thermal analysis results
      1-Bosentan monohydrate; 2-Physical mixture; 3-Magnesium aluminometasilicate, 4-Mg stearate; 5-Croscarmellose sodium
   B- Thermal analysis results
      1-Bosentan monohydrate; 2-Physical mixture; 3-Bosentan monohydrate+Magnesium aluminometasilicate, 4-Bosentan monohydrate+Mg stearate; 5-Bosentan monohydrate+Croscarmellose sodium
   C- Thermal analysis results
      1-Bosentan monohydrate; 2-Physical mixture; 3-Blank SNEDDS: magnesium aluminometasilicate(1.25:1), 4-Bosentan monohydrate loaded liquid SNEDDS: magnesium aluminometasilicate (1.25:1)
**Figure 4****.** XRD diffractogram.
   1-Bosentan monohydrate, 2-Magnesium aluminometasilicate, 3-Blank liquid SNEDDS: magnesium aluminometasilicate (1.25:1), 4-Bosentan monohydrate loaded liquid SNEDDS: magnesium aluminometasilicate (1.25:1)

## Claims

1. Solid Self Nanoemulsifying System tablet comprising bosentan monohydrate, wherein the Bosentan monohydrate containing liquid self nanoemulsifying system is adsorbed on magnesium aluminometasilicate and the liquid self nanoemulsifying system comprises glyceryl monolinoleate as an oil, polyoxyl 40 hydrogenated castor oil as a surfactant and caprylocaproyl polyoxyl-8 glycerides as a co-surfactant.

2. The Solid Self Nanoemulsifying System tablet according to claim 1, wherein the ratio of liquid self nanoemulsifying system components which are glyceryl monolinoleate as an oil, polyoxyl 40 hydrogenated castor oil as a surfactant and caprylocaproyl polyoxyl-8 glycerides as a co-surfactant is 0.9: 7.2: 0.8 (w/w/w) by weight.

3. The Solid Self Nanoemulsifying System tablet according to claim 1, wherein the liquid self nanoemulsifying system particle size is below 50 nm and the polydispersity value is less than 0.2.

4. The Solid Self Nanoemulsifying System tablet according to claim 3, wherein the liquid self nanoemulsifying system particle size is between 10- 50 nm and the polydispersity value is between 0.1-0.2.

5. The Solid Self Nanoemulsifying System tablet according to claim 4, wherein the liquid self nanoemulsifying system particle size is 17.11 nm and the polydispersity index is 0.180.

6. The Solid Self Nanoemulsifying System tablet according to claim 1, wherein 1 gram liquid self nanoemulsifying system comprises 30 mg of Bosentan monohydrate.

7. The Solid Self Nanoemulsifying System tablet according to claim 1, wherein the ratio of liquid self nanoemulsifying system : magnesium aluminometasilicate is between 1:1 and 1.5:1 (w/w) by weight.

8. The Solid Self Nanoemulsifying System tablet according to claim 7, wherein the ratio of liquid self nanoemulsifying system : magnesium aluminometasilicate is 1.25:1 (w/w) by weight.

9. The Solid Self Nanoemulsifying System tablet according to claim 8, wherein it comprises 5% (w/w) croscarmellose sodium and 1% magnesium stearate as excipient.

10. A process for obtaining the Solid Self Nanoemulsifying System tablet comprising Bosentan monohydrate according to claim 9, comprising the steps below:
- Self nanoemulsifying system was obtained with glyceryl monolinoleate as oil, polyoxyl 40 hydrogenated castor oil as a surfactant, and caprilocaproyl polyoxyl-8 glycerides as cosurfactant,
- Bosentan monohydrate was loaded into the self nanoemulsifying system, which consists of glyceryl monolinoleate: Polyoxil 40 hydrogenated castor oil: Caprilocaproyl polyoxyl-8 glycerides.
- Obtaining Solid Self Nanoemulsifying System by loading liquid self nanoemulsifying system on magnesium aluminometasilicate as adsorbent,
- Mixing Solid Self Nanoemulsifying System and tablet excipients,
- Obtaining tablet by direct compression method.

## Patentansprüche

1. Festes Selbstnanoemulgierendes System in Tablettenform, das Bosentan-Monohydrat enthält, wobei das Bosentan-Monohydrat enthaltende flüssige Selbstnanoemulgierende System auf Magnesiumaluminometasilikat adsorbiert ist und das flüssige Selbstnanoemulgierende System Glycerylmonolinoleat als Öl, Polyoxyl-40-hydrogeniertes Rizinusöl als Tensid und Caprylocaproyl-Polyoxyl-8-Glyceride als Co-Tensid umfasst.

2. Das feste Selbstnanoemulgierende System in Tablettenform nach Anspruch 1, wobei das Gewichtsverhältnis der Komponenten des flüssigen Selbstnanoemulgierenden Systems, nämlich Glycerylmonolinoleat als Öl, Polyoxyl-40-hydrogeniertes Rizinusöl als Tensid und Caprylocaproyl-Polyoxyl-8-Glyceride als Co-Tensid, 0,9:7,2:0,8 (G/G/G) beträgt.

3. Das feste Selbstnanoemulgierende System in Tablettenform nach Anspruch 1, wobei die Partikelgröße des flüssigen Selbstnanoemulgierenden Systems unter 50 nm liegt und der Polydispersitätswert weniger als 0,2 ist.

4. Das feste Selbstnanoemulgierende System in Tablettenform nach Anspruch 3, wobei die Partikelgröße des flüssigen Selbstnanoemulgierenden Systems zwischen 10-50 nm liegt und der Polydispersitätswert zwischen 0,1 und 0,2 liegt.

5. Das feste Selbstnanoemulgierende System in Tablettenform nach Anspruch 4, wobei die Partikelgröße des flüssigen Selbstnanoemulgierenden Systems 17,11 nm beträgt und der Polydispersitätsindex 0,180 beträgt.

6. Das feste Selbstnanoemulgierende System in Tablettenform nach Anspruch 1, wobei 1 Gramm des flüssigen Selbstnanoemulgierenden Systems 30 mg Bosentan-Monohydrat enthält.

7. Das feste Selbstnanoemulgierende System in Tablettenform nach Anspruch 1, wobei das Gewichtsverhältnis des flüssigen Selbstnanoemulgierenden Systems zu Magnesiumaluminometasilikat zwischen 1:1 und 1,5:1 (G/G) liegt.

8. Das feste Selbstnanoemulgierende System in Tablettenform nach Anspruch 7, wobei das Gewichtsverhältnis des flüssigen Selbstnanoemulgierenden Systems zu Magnesiumaluminometasilikat 1,25:1 (G/G) beträgt.

9. Das feste Selbstnanoemulgierende System in Tablettenform nach Anspruch 8, das 5% (G/G) Croscarmellose-Natrium und 1% Magnesiumstearat als Hilfsstoffe enthält.

10. Ein Verfahren zur Herstellung der festen Selbstnanoemulgierenden System-Tablette, die Bosentan-Monohydrat nach Anspruch 9 enthält, umfassend die folgenden Schritte:
- Das Selbstnanoemulgierende System wurde mit Glycerylmonolinoleat als Öl, Polyoxyl-40-hydrogeniertem Rizinusöl als Tensid und Caprylocaproyl-Polyoxyl-8-Glyceriden als Co-Tensid hergestellt.
- Bosentan-Monohydrat wurde in das aus Glycerylmonolinoleat bestehende selbstemulgierende System geladen: Polyoxil 40 hydriertes Rizinusöl: Caprilocaproyl Polyoxyl-8 Glyceride.
- Gewinnung des festen Selbstnanoemulgierenden Systems durch Beladung des flüssigen Selbstnanoemulgierenden Systems auf Magnesiumaluminometasilikat als Adsorbens.
- Mischen des festen Selbstnanoemulgierenden Systems mit Tablettenhilfsstoffen.
- Herstellung der Tablette durch Direktkompressionsverfahren.

## Revendications

1. Comprimé solide à système auto-émulsifiant comprenant du bosentan monohydraté, dans lequel le bosentan monohydraté contenant un système liquide auto-émulsifiant est adsorbé sur de l'aluminometasilicate de magnésium et le système liquide auto-émulsifiant comprend du monolinoléate de glycéryle comme huile, de l'huile de ricin hydrogénée polyoxyl 40 comme tensioactif et des glycérides de caprylocaproyl polyoxyl-8 comme co-tensioactif.

2. Comprimé de système solide auto-émulsifiant selon la revendication 1, dans lequel le rapport des composants du système liquide auto-émulsifiant qui sont le monolinoléate de glycéryle en tant qu'huile, l'huile de ricin hydrogénée polyoxyl 40 en tant qu'agent tensioactif et les glycérides caprylocaproyl polyoxyl-8 en tant que co-agent tensioactif est de 0,9:7,2 : 0,8 (p/p/p) en poids.

3. Comprimé de système solide auto-émulsifiant selon la revendication 1, dans lequel la taille des particules du système liquide auto-émulsifiant est inférieure à 50 m et la valeur de polydispersité est inférieure à 0,2.

4. Comprimé de système solide auto-émulsifiant selon la revendication 3, dans lequel la taille des particules du système liquide auto-émulsifiant est comprise entre 10 et 50 nm et la valeur de polydispersité est comprise entre 0,1 et 0,2.

5. Comprimé de système solide auto-émulsifiant selon la revendication 4, dans lequel la taille des particules du système liquide auto-émulsifiant est de 17,11 nm et l'indice de polydispersité est de 0,180.

6. Comprimé de système solide auto-émulsifiant selon la revendication 1, dans lequel 1 gramme de système liquide auto-émulsifiant comprend 30 mg de bosentan monohydraté.

7. Comprimé de système solide auto-émulsifiant selon la revendication 1, dans lequel le rapport entre le système liquide auto-émulsifiant et l'aluminométasilicate de magnésium est compris entre 1:1 et 1,5:1 (p/p) en poids.

8. Comprimé de système solide auto-émulsifiant selon la revendication 7, dans lequel le rapport entre le système liquide auto-émulsifiant et l'aluminométasilicate de magnésium est de 1,25:1 (p/p) en poids.

9. Comprimé de système solide auto-émulsifiant selon la revendication 8, dans lequel il comprend 5 % (p/p) de croscarmellose sodique et 1 % de stéarate de magnésium en tant qu'excipient.

10. Procédé d'obtention du comprimé de système solide auto-émulsifiant comprenant le bosentan monohydraté selon la revendication 9, comprenant les étapes suivantes :
- Un système auto-nanoémulsifiant a été obtenu avec du monolinoléate de glycéryle comme huile, de l'huile de ricin hydrogénée polyoxyl 40 comme tensioactif, et des glycérides caprilocaproyl polyoxyl-8 comme co-tensioactif.
- Le bosentan monohydraté a été chargé dans le système auto-nanoémulsifiant, qui se compose de monolinoléate de glycéryle : huile de ricin hydrogénée Polyoxil 40 : Caprilocaproyl polyoxyl-8 glycérides.
- Obtention d'un système auto-émulsifiant solide par chargement du système auto-émulsifiant liquide sur de l'aluminometasilicate de magnésium en tant qu'adsorbant,
- Mélange du système solide auto-émulsionnant et des excipients du comprimé,
- Obtention du comprimé par la méthode de compression directe.
